# EUROPEAN PATENT APPLICATION

(11) **EP 2 138 122 A1**
(43) Date of publication of application: **30.12.2009**
(21) Application number: 08305326.4
(22) Date of filing: 25.06.2008
(51) Int. Cl.: A61B 17/70

(54) **Stabilization system between a sacrum and a lumbar vertebra**

(71) Applicant: Abbott Spine, La Cité Mondiale, 33000 Bordeaux (FR)
(72) Inventor: Douget, Stéphane, 33110 Le Bouscat (FR); Pointillart, Vincent, 33000 Bordeaux (FR)
(74) Representative: Dronne, Guy

(57) **Abstract**

The invention concerns a stabilization system between a sacrum and a lumbar vertebra. It comprises:
two anchors (12, 14) suitable for being fastened in the sacrum (S);
a braid system (16, 18) presenting at least one free end (16a, 18a) to enable traction to be exerted thereon, and co-operating mechanically with the spinous process of said vertebra; and
at least one blocking system (20) for blocking said free end of the braid after said traction has been exerted;
said braid system (16, 18) and said blocking system (20) co-operating with each other and with said anchors (12, 14) to provide two traction assemblies between said spinous process (A) and said anchors (12, 14).

## Description

The present invention provides a system for providing stabilization, optionally dynamic stabilization, between the sacrum and at least one lumbar vertebra.

Numerous documents are known that describe systems for stabilizing the sacrum with at least the fifth lumbar vertebra. Such systems are described in particular by PCT patent applications Nos. WO 2005/020860, WO 2006/106268, and WO 2007/023240 in the name of Abbott Spine.

Those devices are very effective, however they prevent the sacrum from moving relative to the vertebra(e) in a manner that is almost total, thereby leaving no degree of freedom between the sacrum and the last lumbar vertebra.

In a large number of cases, such a situation is unfavorable. The surgeon desires to leave the possibility of a reduced amount of relative movement between the sacrum and the last lumbar vertebra.

In general, in systems for stabilizing the sacrum with at least one lumbar vertebra, use is made of screws of the pedicular screw type that are fastened in the sacrum. Reference can be made to above-mentioned PCT patent applications WO 2007/023240 or WO 2006/106268. In certain clinical situations, the use of such screws is not very desirable. In particular, the screw heads make the postoperative stage more complicated. In addition, in certain kinds of deterioration of the sacrum, pedicular type screws cannot be anchored in the sacrum sufficiently securely for them to be able to transfer high levels of traction.

To solve those problems, an object of the present invention is to provide a system for stabilizing the sacrum with at least the fifth lumbar vertebra, which system makes dynamic stabilization possible and achieves better fastening in the sacrum.

In the particular circumstances under consideration, the term "dynamic stabilization" should be understood as meaning that the sacrum and at least the fifth lumbar vertebra are indeed stabilized relative to each other, while leaving the possibility for a limited amount of relative movement between the vertebra and the sacrum so as to give greater comfort to the patient.

According to the invention, this object is achieved by an optionally dynamic stabilization system between the sacrum and at least one lumbar vertebra, which system is **characterized in that** it comprises:
· two anchors suitable for being fastened in the sacrum;
· a braid system presenting at least one free end to enable traction to be exerted thereon, and co-operating mechanically with the spinous process of said vertebra; and
· at least one blocking system for blocking said free end of the braid after said traction has been exerted;
said braid system and said blocking system co-operating with each other and with said anchors to provide two traction assemblies between said spinous process and said anchors.

The term "co-operate" should be understood as meaning that the braid is not necessarily in direct contact with the process, and that it may be mechanically connected thereto via a mechanical part in order to provide stabilization under all circumstances.

It will be understood that by virtue of the provisions of the invention, the effectiveness with which the ends of the braid are fastened to the sacrum is improved by using anchors, where such anchors are also in more widespread use than are pedicular screw type fastener systems, in particular for other clinical purposes.

In addition, the blocking system that serves to exert traction on the two braid ends provides appropriate stabilization and makes it possible to remedy the fact that, with anchors, it is not possible for the surgeon to exert traction on the ends of the braid prior to fastening them to the anchors, since the anchor itself does not enable a braid end to be blocked relative thereto after traction has been exerted thereon.

In a first configuration, said braid system comprises two braids, a first end of each braid is secured to one of said anchors, and the second end of each braid forms a free traction end engaged in said blocking system.

Under such circumstances, the blocking system is a component that is independent from the anchors.

In a first embodiment of the first configuration, said blocking system comprises a body presenting two end faces, with two orifices formed in said body and opening out into the two faces, each orifice being suitable for receiving a respective end of the first and second braid elements, and two movable blocking members suitable for being inserted in the ends of slots opening out into said faces, whereby the braid ends are blocked in the body of the blocking system by wedging.

In a second embodiment of the first configuration, the blocking system comprises a body provided with at least one orifice passing right through it, said orifice being suitable for receiving one end of each braid element, said orifice including a shoulder forming a clamping face, and a blocking member that is movable in said orifice, one end of said blocking member forming a second clamping face, the ends of the braid elements being disposed between said clamping faces.

Preferably, the complete stabilization system also includes a spacer interposed between the spinous process of the fifth lumbar vertebra L5 and spinous process S1 of the sacrum S.

Under such circumstances, the blocking system may be a component that is separate from the spacer, or it may be mounted on the spacer.

In a second configuration, the braid system comprises a single braid, said blocking system is mechanically connected to one of said anchors, said blocking system co-operating with the free end of the braid, and the second end of the braid is secured to the second anchor.

In a variant of the second configuration, the braid system comprises a single braid, the stabilization system comprises two blocking systems, each blocking system receiving a free end of said braid, and each blocking system is mechanically connected to an anchor.

In a first embodiment of the second configuration, each blocking system is a self-blocking system serving to block the braid under the effect of the traction exerted on the free end of the braid.

In a second embodiment of the second configuration, each blocking system comprises a body that is secured to the head of the anchor by a screw fastener member.

Other characteristics and advantages of the invention appear better on reading the following description of a plurality of embodiments of the invention given as non-limiting examples. The description refers to the accompanying figures, in which:
· Figure 1 is a view showing the principle of the stabilization system in a first configuration;
· Figure 2A is a perspective view of a first embodiment of the blocking system for the first configuration;
· Figure 2B is a vertical section view of the Figure 2A blocking system;
· Figure 2C is a perspective view of a variant embodiment of the Figure 2A blocking system;
· Figure 3A is a perspective view of a second embodiment of the blocking system for the first configuration;
· Figure 3B is a vertical section view of the Figure 3A blocking system;
· Figure 3C is a horizontal section view of the Figure 3A blocking system;
· Figures 4 and 5 show examples of anchors that can be used in the stabilization system;
· Figure 6 shows a complete stabilization system including a spinous spacer;
· Figure 7 shows a stabilization system for stabilizing the sacrum and two lumbar vertebrae;
· Figure 8 is a rear view of a first variant of the stabilization system for stabilizing the sacrum and one lumbar vertebra;
· Figure 8A is a side view of the Figure 8 stabilization system;
· Figure 8B is a detail view of Figure 8;
· Figure 9 shows a second variant of the system for stabilizing the sacrum and one lumbar vertebra;
· Figure 9A is a detail view of the Figure 9 stabilization system;
· Figure 10 shows a third variant of the system for stabilizing the sacrum and two lumbar vertebrae;
· Figure 10A is a detail view of the Figure 10 stabilization system;
· Figure 11 is a perspective view of a second configuration of the stabilization system;
· Figure 12 is an exploded view of a preferred embodiment of the blocking system shown in Figure 1;
· Figure 12A is a section view on line A-A of Figure 12;
· Figure 13 is an exploded view, partially in section, showing a variant embodiment of the means for fastening the blocking system to the sacrum;
· Figure 14 is a longitudinal section view of the means for blocking on the sacrum with the help of the fastener means shown in Figure 13;
· Figure 15 is a perspective view of a variant embodiment of the stabilization system shown in Figure 11; and
· Figure 15A is a fragmentary view of Figure 15 on line A-A.

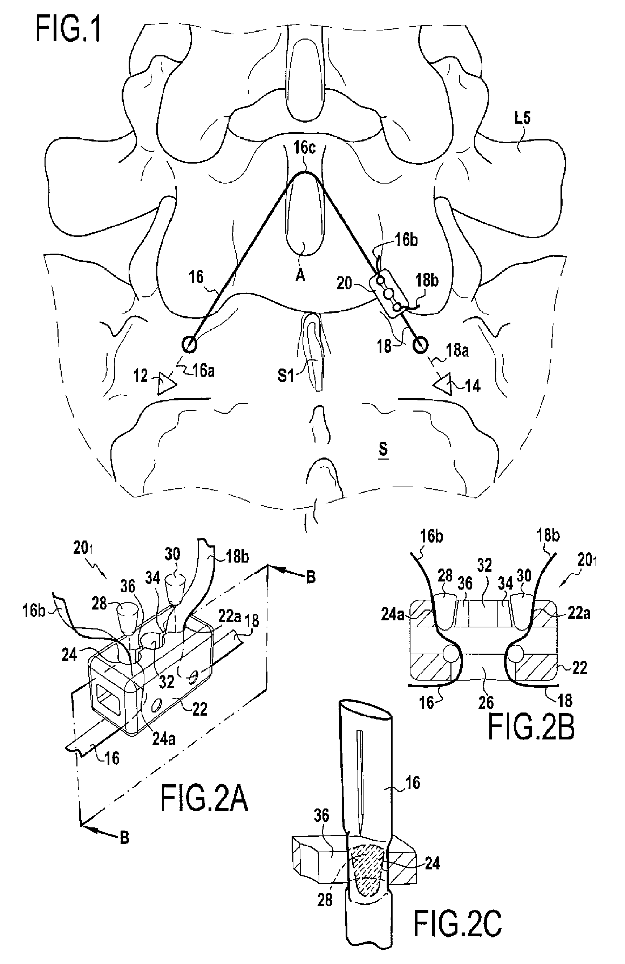

With reference initially to Figure 1, there follows a description of the essential elements of the dynamic stabilization system in the first configuration that enables the sacrum and lumbar vertebra L5 to be stabilized.

Figure 1 shows the sacrum S with its spinous process S1, together with lumbar vertebra L5 with its spinous process referenced A. The stabilization system, preferably a dynamic system, comprises two anchor means constituted by anchors referenced 12 and 14 that are put into place in the sacrum at suitable locations. The system also comprises a braid made of flexible material and referenced 16, which braid is constituted, in fact, by two braid elements given respective references 18 and 20. The stabilization system also has a blocking system 20.

The first braid element 18 has a first end 18a that is fastened to the anchor 14 and a free second end 18b that is engaged in the blocking system 20. The second braid element 16 presents a first end 16a that is attached to the anchor 12, and a free second end 16b that is engaged in the blocking system 20. An intermediate portion 16c of the braid element 16 passes over the top portion of the spinous process A of the lumbar vertebra L5. As explained in greater detail below, prior to blocking in the blocking system 20, the surgeon can exert traction on the free ends 16b and 18b of the two braid elements in order to obtain the desired stabilization effect by adapting the strength of the traction that is applied to the braid element. Once the desired stabilization effect has been obtained by applying appropriate traction, the surgeon blocks the ends of the braids in the blocking system, as explained below.

One of the advantages of the invention lies in using anchors for fastening the ends 16a and 18a of the braid elements to the sacrum. Anchors are conventionally used in the field of surgical repair, e.g. for fastening artificial ligaments to bones in order to enable natural ligaments to be repaired. Anchors generally achieve more reliable anchoring in the bone, as explained below, because such anchors can include systems that project into the bone, and furthermore such anchors are much more compact than are screws of the pedicular type as are conventionally used.

It is clear that fastening the ends of the braid elements directly to the heads of the anchors does not enable the tension in the braid elements 16 and 18 to be adjusted, as can be done when using screws of the pedicular screw type. Nevertheless, this situation does not constitute any kind of drawback, since the presence of the blocking system 20 makes it easy to exert said traction prior to blocking the ends of the braid elements.

In addition, since the braid elements are preferably made out of a material that is flexible and that optionally presents a certain amount of elasticity, they can retain the possibility of a limited amount of relative movement between the sacrum and the vertebra.

With reference now to Figures 2A and 2B, there follows a description of a first embodiment of the blocking system, given reference 20₁. This blocking system comprises a body 22 that is substantially in the form of a rectangular parallelepiped, presenting on its top face 22a two lateral orifices 24 that communicate, for example, with a single orifice 26 made in the bottom face 22b of the body. The ends 24 of the orifices are generally cylindrical in shape. The ends of the braid elements 16 and 18 can be inserted into the body 22 via the orifices 24, and they can leave it by the orifice 26. Wedge-shaped blocking members 28 and 30 can be engaged in the ends of the orifices 24 in order to block the braid elements 16 and 18 by being wedged between the wedges 28 and 30 and the inside walls 24a of the orifices 24. By way of example, the surgeon begins by exerting traction on the ends 16a and 18b of the braid elements, and then once an appropriate level of traction is being exerted, the surgeon puts the blocking members 28 and 30 into place to hold the ends of the braid elements securely.

Figure 2C shows a variant embodiment of the blocking system 20₁. The braid is of tubular shape and the wedge-shaped element 28 is located inside the braid. The member 28 can be put into place through a slot formed in the tubular braid 16 and not shown in the figure. When appropriate traction has been exerted on the end of the braid element with the help of a suitable instrument, the surgeon can cause the blocking member 28 to move so as to cause said member to co-operate with the top end of the orifice 24. This produces a very good wedging effect on the braid because the braid is interposed between the inside wall 24a of the orifice 24 and the wedge-shaped part 28 over 360 degrees.

It is also possible to provide a central orifice 32 in the top face 22a of the body 22, the central orifice extending the bottom orifice 26. This orifice is of dimensions greater than the orifices 24 and enables the braid elements 16 and 18 to be put into place initially in the body 22, after which the braid elements are taken into the orifices 24 via slots 34 and 36 that put these orifices 24 into communication with the central orifice 32.

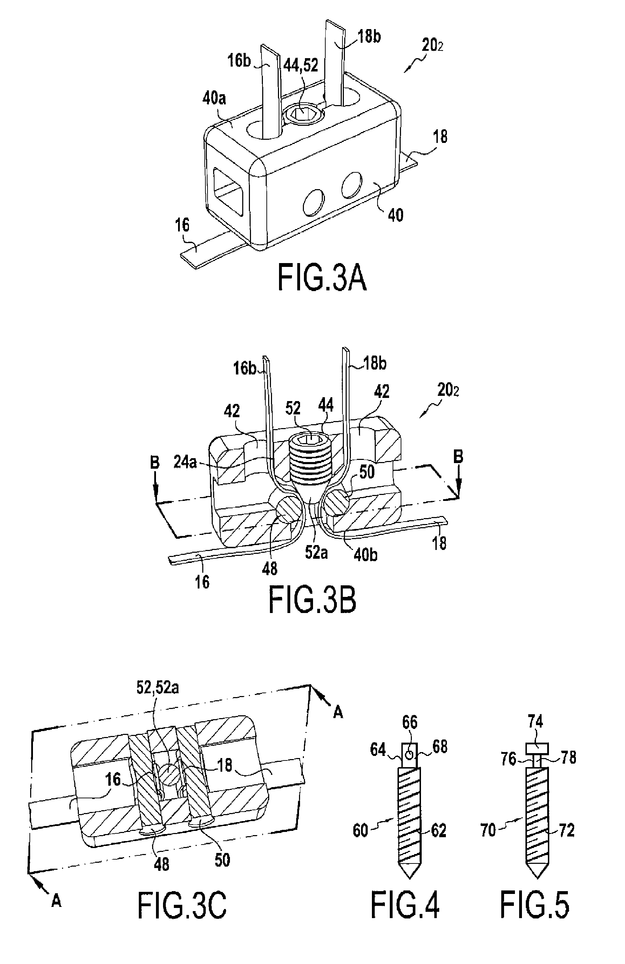

With reference to Figures 3A, 3B, and 3C, there follows a description of a second embodiment of the blocking system 20₂. The blocking system comprises a body 40 having two orifices 42 opening out into its top face 40a. The top face 40a also includes a central orifice 44 that is provided with tapping 44a. The body 40 also has an axial orifice 46 in its bottom face 40b, which axial orifice 46 communicates with the top orifices 42. Diabolo-shaped parts such as 48 and 50 are mounted on either side of the bottom orifice 46 to form clamping surfaces. As shown in Figure 3A, the braid elements 16 and 18 penetrate into the body of the blocking system via the bottom orifice 46, passing over the clamping surfaces 48 and 50. The braid elements 16 and 18 leave via the top orifices 42. In order to block the braid elements, a screw 52 co-operates with the tapping 44a in the central orifice 44. The bottom end 52a of the screw 52 acts, when the screw 52 is tightened, to clamp portions of the braid elements 16 and 18 between said end 52a and the clamping surfaces 50 and 48. As in the first embodiment, the surgeon begins by exerting appropriate traction on the ends 16b and 18b of the braid elements and then, by acting on the screw 52, the ends of the braid elements are blocked relative to the blocking system 20₂.

Naturally, other blocking systems could be used instead of the system 20₁ or 20₂, providing such systems make it possible initially to exert traction on the ends of the braid elements, prior to blocking them relative to the blocking system.

In particular, and as explained below, the blocking system may form part of an intervertebral spacer that is used in the stabilization system.

Anchors are commonly used in certain fields of surgery for enabling ligaments to be fastened to bones, and in particular artificial ligaments, specifically when performing surgery on joints.

There exist numerous types of anchor. Accompanying Figures 4 and 5 show two examples, with Figure 4 showing an anchor 60 comprising a threaded body 62 and a head 64 of small dimensions including a hole 66 for attaching one end of a braid 68.

In the embodiment of Figure 5, the anchor 70 naturally likewise has a threaded body 72 and a head 74, which head is disk-shaped and presents a groove 76. The groove 76 enables the end of a ligament or of a braid 78 to be attached thereto.

Other types of anchor that are likewise suitable for use in dynamic stabilizer systems of the present invention are provided with expandable anchor members that are moved, after the anchor has been screwed into the bone, to penetrate into the bone by projecting out from the threaded body of the anchor.

Anchors of this type are described for example in the following patents: EP 0 558 993; EP 0 619 982; EP 0 668 055; and WO 96/09798.

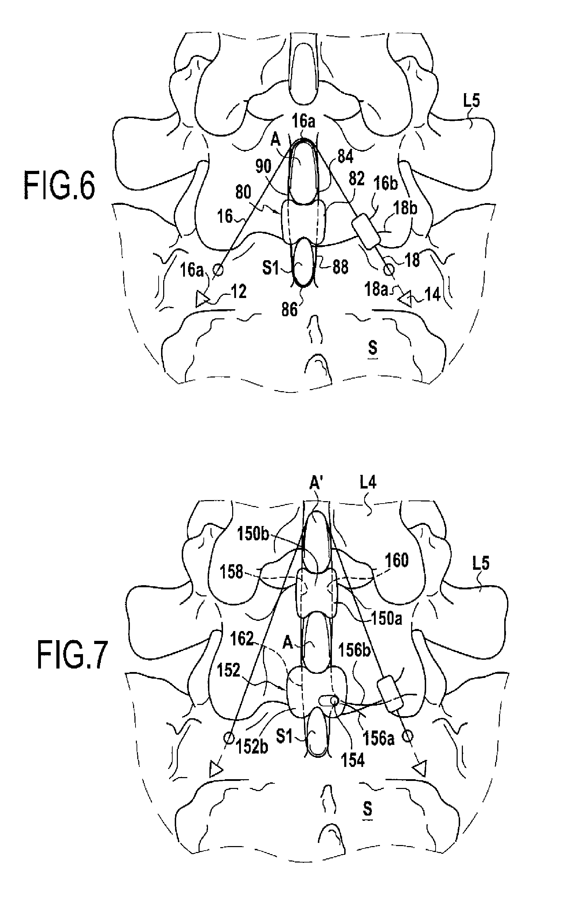

With reference below to Figures 6 to 10, there follows a description of complete embodiments of the optionally-dynamic first configuration of the stabilization system, including at least one interspinous spacer.

Figure 6 shows a dynamic stabilization system comprising all of the component elements of the embodiment of Figure 1 together with an interspinous spacer 80.

The spacer 80 comprises a body 82 provided at both ends with respective housings 84 and 86 for receiving the spinous processes A and S1. The spacer 80 also has two fastener ties 88 and 90 for holding the body 82 of the spacer in place between the processes.

Advantageously, this spacer is of the type described in the PCT patent applications in the name of Abbott Spine and numbered WO 01/28442; WO 02/03882; or indeed WO 02/051326.

The function of the spacer 80 is to provide stabilization when the two processes move towards each other, the braid elements 16 and 18 serving to provide dynamic stabilization when the spinous processes move apart from each other.

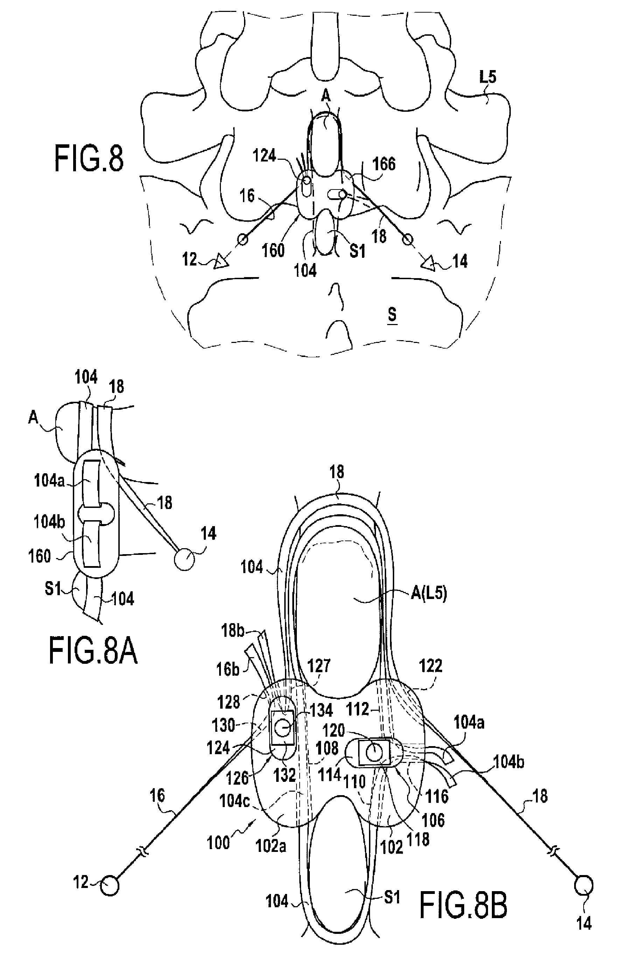

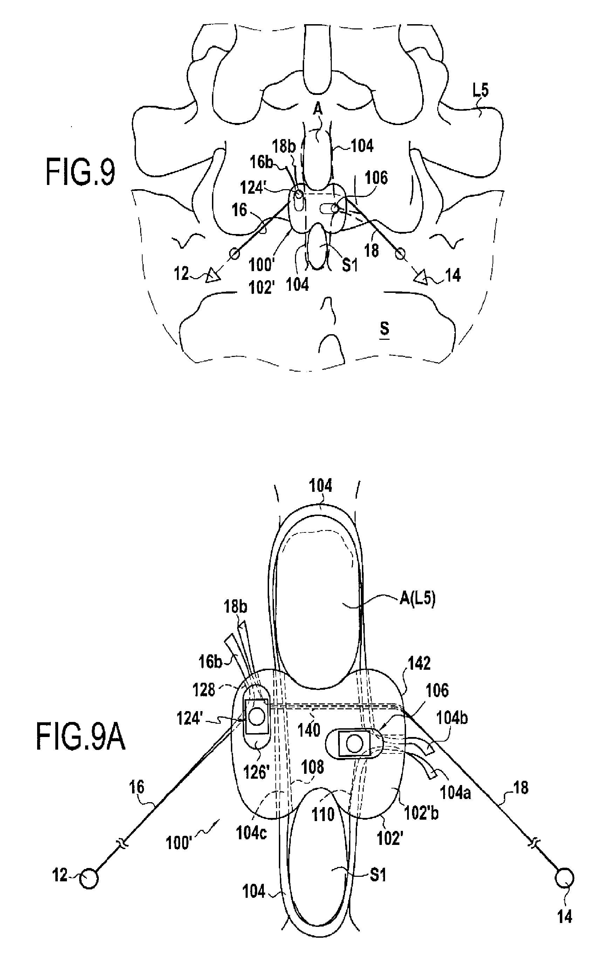

In Figures 8 and 9, there can be seen two other embodiments of the stabilization system between the sacrum and lumbar vertebra L5. In these embodiments, the blocking system is mounted directly on the spacer that is interposed between S1 and L5.

In Figure 8, the spacer is referenced 100. It comprises a body 102 and a braid or tie 104 for fastening to the processes A and Sl, together with a system 106 for securing the free ends 104a and 104b of the braid 104.

As shown better in Figure 8B, the middle portion 104c of the braid 104 passes through the spacer body 102 via a passage 108 that is formed in the distal portion 102a of the spacer body; it passes over the processes A and 51 and penetrates into the spacer body via internal passages 110 and 112 that open out into an internal housing 114 that forms a portion of the system 106 for securing the braid to the spacer body. The free ends 104a and 104b of the braid exit the spacer body via an internal exit passage 116. In the internal housing 114 there is mounted a part 118 that is movable in translation and that can be moved with the help of a control screw 120. In a first position of the part 118, the portions of the braid 104 are free in the housing 114. In a second position, these portions of the braid are clamped between the proximal face of the moving portion 118 and the inside wall of the housing 114, thereby securing the ends 104a and 104b of the braid to the spacer body 102.

The braid element 18 fastened to the anchor 14 passes through the top end of the proximal portion 102b of the spacer body via an internal passage 122, and then passes over the process A of vertebra L5. Its free second end 18b co-operates with the blocking system 124 that forms an integral portion of the spacer body 102. The braid element 18 penetrates into an internal housing 126 of the blocking system 124 via an internal passage 127, and its end 18b exits therefrom via the passage 128.

The braid element 16 has one end 16a fastened on the anchor 12 and penetrates into the distal portion 102a of the spacer body via internal passage 130 that opens out into the housing 126, The end 16b of the spacer element exits via the internal passage 128. The blocking system 24 is very similar to the securing system 106 and comprises, in addition to the housing 126, a part 132 that is movable in translation in the housing 126, with movements thereof being controlled by the screw 134. At rest, the moving part 132 can occupy a first position in which the portions of the braid element placed in the housing 126 are free. The part 132 can also occupy a second position in which the portions of the braid elements 16 and 18 that are located in the housing 126 of the blocking system 124 are pinched or clamped between one end of the moving part 132 and a portion of the side wall of the housing 126 close to the passage 128. In this position, the ends 16b and 18b of the braid elements 16 and 18 are blocked in the spacer body 102.

Figures 9 and 9A show another embodiment of the dynamic stabilization system for stabilizing the sacrum and lumbar vertebra L5.

It differs from the embodiment of Figures 8, 8A, and 8B essentially by the fact that the braid element 18 does not pass over the process A of vertebra L5, but co-operates mechanically therewith.

The spacer, referenced 100', comprises a spacer body 102', a braid 104 for fastening the spacer body to the processes, and the system 106 for securing the braid. The spacer body 102' is fastened on the spinous processes A and S1 with the help of the braid 104 in a manner identical to that used by the spacer body 102 in Figures 8, 8A, and 8B. It is therefore not described again.

The stabilization system includes a blocking system 124' for blocking the ends 16b and 18b of the braid elements, which system is identical to the blocking system 124 of Figures 8, 8A, and 8B. It is therefore not described again.

In a variant, the blocking system 106 could be replaced by a dual self-blocking device of the type described in PCT patent application WO 2005/120277 in the name of Abbott Spine. Under such circumstances, the blocking system would be clipped to the spacer body.

The braid element 18 penetrates into the spacer body 102' via an internal passage 140 that opens out into the outer face 142 of the proximal end 102'b of the spacer body and into the housing 126 of the blocking system 124'. It thus passes into the housing 126 of the blocking system 124', and its ends 18b exits therefrom via the internal passage 128. The braid element 16 penetrates into the housing 126 via the internal passage 130, and its end 16b exits therefrom via the internal passage 128.

By moving the moving part 132 of the blocking system 124' with the help of the screw 134, it is possible to block the ends 16b and 18b of the braid elements 16 and 18 against the spacer body 102'.

Figure 7 shows a first embodiment of the stabilization system for stabilizing the sacrum S with lumbar vertebrae L5 and L4.

The stabilization system comprises a partial subsystem that is identical to that shown in Figure 1. The partial subsystem comprises anchors 12 and 14, braid elements 16 and 18, and the blocking system 20. The only difference is that the braid element 16 passes over the spinous process A' of vertebra L4 and not over process A of vertebra L5.

The stabilization system further comprises a single spacer 150 interposed between the processes A and A', and a spacer 152 provided with a fastener system 154 that is interposed between the processes A and S1 of vertebra L5 and of the sacrum S, together with a fastener braid or tie 156.

The fastener braid 156 passes freely through the spacer 150 via two internal passages 158 and 160 formed in the proximal and distal ends 150a and 150b of the spacer 150.

In its distal portion 152b, the spacer 152 includes an internal passage 162, and the fastener system 154 is made in its proximal portion 152a.

The fastener braid 156 passes over the spinous processes A' of vertebra L4 and S1 of the sacrum S.

The braid passes through the spacer 150 via the passages 158 and 160, and through the spacer 152 via the passage 162. The ends 156a and 156b of the fastener braid 156 co-operate with the securing system 154.

It can be understood that the braid elements 16 and 18 provide dynamic stabilization of the two vertebrae and of the sacrum when these parts of the anatomy tend to move apart while the assembly constituted by the spacers 150 and 152 and the fastener braid 156 serve to provide stabilization when the same parts of the anatomy tend to move towards one another.

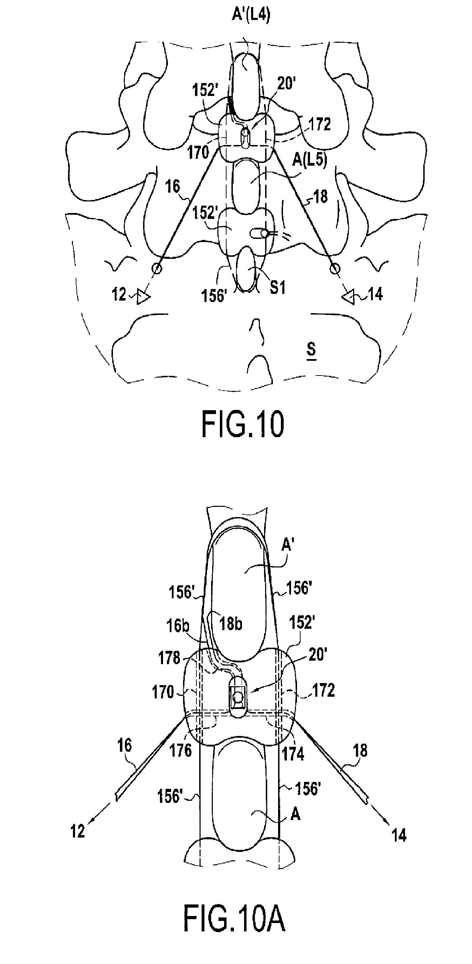

In Figures 10 and 10A, there can be seen a variant of the stabilization system that is optionally dynamic and that serves to stabilize lumbar vertebrae L5 and L4 with the sacrum S.

The system comprises a subassembly constituted by a spacer 150' interposed between the spinous processes A and A', a spacer 152' identical to the spacer 152 of Figure 7, and a fastener braid 156' identical to that of Figure 7. This subassembly serves to stabilize the two vertebrae and the sacrum when these parts of the anatomy tend to move towards one another.

The second subassembly constituting the stabilization system of Figures 10 and 10A differs from that of Figure 7 by the fact that the blocking system 20' is not independent, but is mounted on the spacer 150'.

The braid elements 16 and 18 have their ends 16a and 16b fastened to the anchors 12 and 14, and they penetrate into the body of the spacer 150' via internal passages 170 and 172 that open out into the blocking system 20' for blocking the ends 16b and 18b of the braid elements 16 and 18.

The blocking system 20' is identical to the blocking system 124 shown in Figures 8B or 9A.

The only difference lies in the fact that the blocking system preferably occupies a central position relative to the spacer 150'. The braid elements 16 and 18 penetrate into the blocking system 20 via the internal passages 174 and 176, and the ends 16b and 18b of these braid elements exit therefrom via the internal passage 178.

In all of the embodiments described with reference to Figures 6 to 10, the dynamic stabilization braid elements 16 and 18 co-operate directly or indirectly (via a spacer) with the fifth or the fourth lumbar vertebra.

In any event, by exerting appropriate traction on the ends 16b and 18b of the braid elements, the surgeon can adapt the amount of dynamism imparted to the stabilization in combination with the flexibility and possibly the elasticity of the braid or tie elements. This adaptation serves to define the amplitude of relative movement that is possible between the sacrum and the lumbar vertebra(e) during relative movement between these parts of the anatomy when they tend to move apart from one another.

Advantageously, the traction can be exerted with the help of an instrument for tensioning a flexible tie and of the type described in PCT patent application WO 2007/034112 in the name of Abbott Spine.

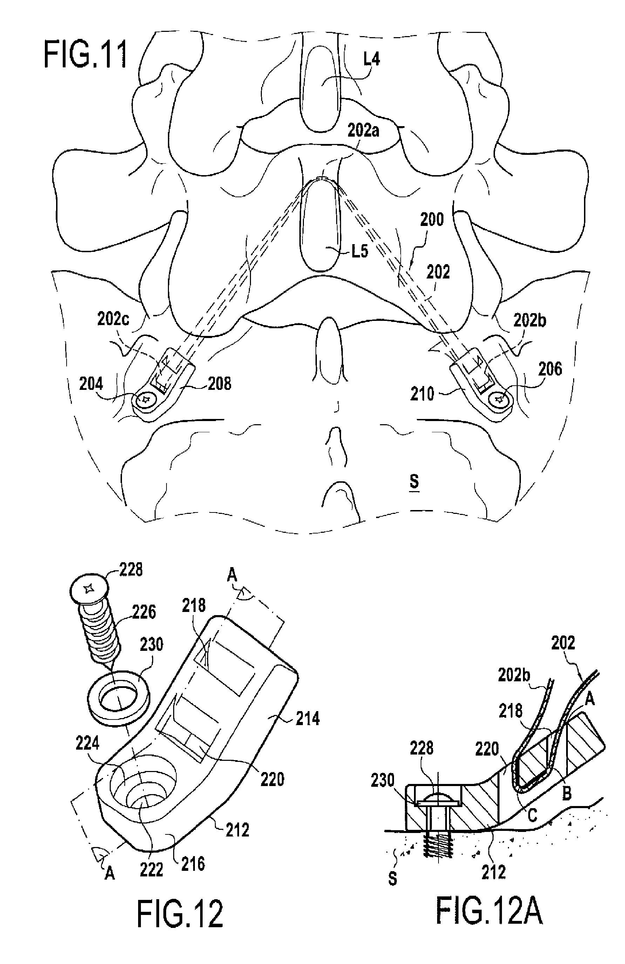

With reference below to Figures 11 to 15, there follows a description of a second configuration of the stabilization system, which configuration can optionally be dynamic.

Figure 11 shows the sacrum S and the spinous processes L5 and L4 of the stabilization system 200 for stabilizing the sacrum and lumbar vertebra L5.

The stabilization system comprises a braid 202, two anchors 204 and 206 fastened to the sacrum, and two blocking systems 208 and 210 that are mechanically connected directly to the anchors, as explained below with reference to Figures 12, 12A, 13, and 14.

The middle portion 202a of the braid 202 passes over the spinous process of vertebra L5, and its free ends 202b and 202c are engaged in the blocking systems 208 and 210 that are secured to the sacrum by the anchors 204 and 206.

In the example shown in Figures 12 and 12A, the blocking system 208 (210) is of the self-blocking type. It comprises a body 212 having a blocking portion 214 and a fastener portion 216.

The blocking portion 214 has two slots 218 and 220 that are inclined and that define friction edges A, B, and C. The braid 202 is engaged in these slots 218 and 220. By exerting traction on the free end 202b of the braid, the braid is blocked relative to the body 212 of the blocking system.

The fastener portion 216 includes a hole 222 that terminates in a circular counterbore 224. The anchor 226 is engaged in the hole 222 and its head constitutes a disk-shaped shoulder 228. By placing a washer 230 in the counterbore 224, it is possible to fasten the blocking system 208 (210) to the sacrum S.

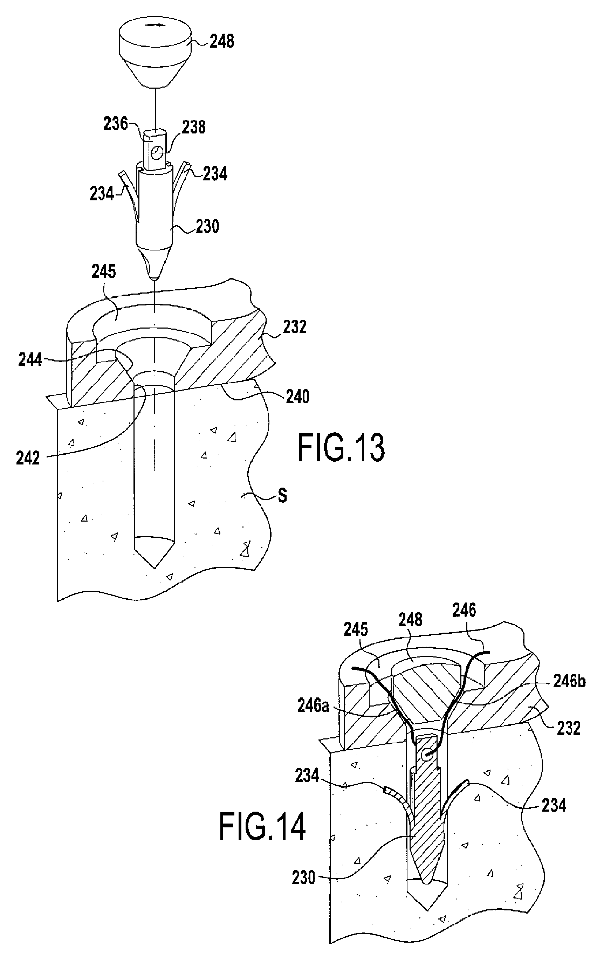

Figures 13 and 14 show a variant mechanical connection between the anchor 230 and the blocking system 232.

The anchor 230 is of the expansion type. It includes expandable members 234 that penetrate into the sacrum when traction is exerted on the head of the anchor.

The anchor 230 also has a flat head 206 provided with an orifice 238 that allows a filamentary element to be passed therethrough.

The blocking system is referenced 232 and it includes a blocking portion identical to that of Figure 12 together with a fastener portion 240. The fastener portion 240 is provided with an orifice 242 extended by a frustoconical portion 244 and a counterbore 245. To fasten the blocking system 232 on the sacrum, the anchor 230 fitted with the filamentary elements 246 engaged in the orifice 238 of the anchor is put into place. By exerting traction on the filamentary element 246, the extensions 234 are caused to become anchored in the sacrum. The filamentary element 246 has both ends passing through the orifice 242, the frustoconical portion 244, and the counterbore 245. A part 248 in the form of a frustoconical wedge is put into place in the frustoconical portion 244 of the orifice, in such a manner that a portion 246a, 246b of each strand of the filamentary element 246 is located between the wedge 248 and the wall of the frustoconical portion 244. After appropriate traction has been exerted on the ends of the filamentary element 246, the wedge 248 is forced into the frustoconical portion 246, thereby mechanically securing the blocking system 232 with the anchor 230.

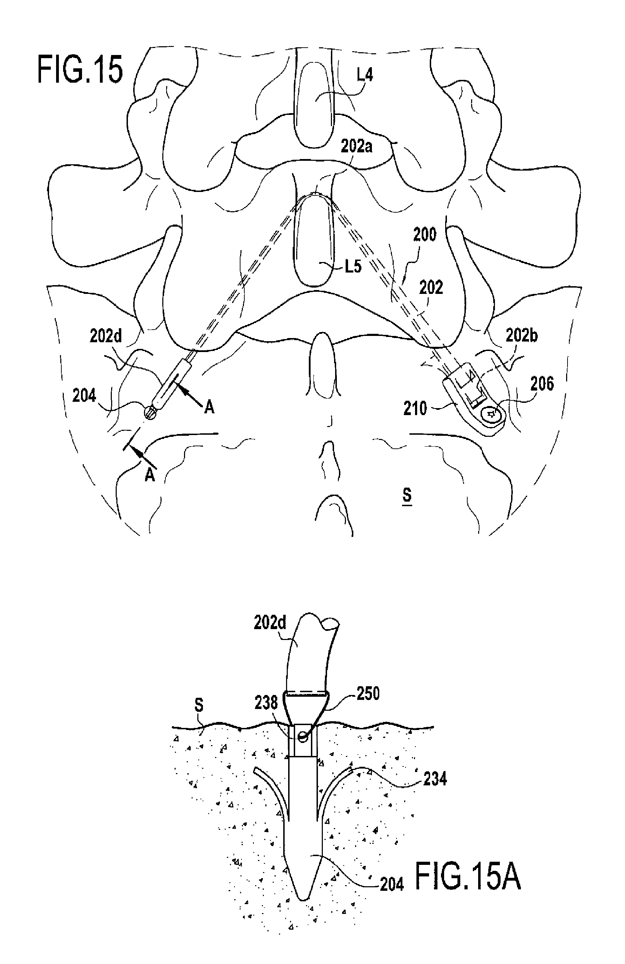

Figures 15 and 15A show a variant of the embodiment of Figure 11. The braid 202 that passes over the spinous process of lumbar vertebra L5 has a free first end 202b that is engaged in a blocking system 210 identical to that of Figure 11. The blocking system 210 is secured to the anchor 206. The other end 202d of the braid 202 is connected directly to the second anchor 206.

Figure 15A shows an embodiment of the connection between the anchor 204 and the end 202d of the braid 202. This direct mechanical connection can be made with the help of a ligament 250 passing through the orifice 238 of the anchor head 204 and passing in a loop made at the end 202d of the braid 202. Traction can then be applied to the braid 202, but only from one end 202b of the braid.

It is also possible to make provision for the anchor and the blocking system to constitute a single part.

Similarly, instead of using anchors with a threaded body or anchors that include elements that can be expanded into the sacrum, it is possible to use impaction anchors of the staple type.

## Claims

1. A stabilization system between a sacrum and a lumbar vertebra, the system being **characterized in that** it comprises:
· two anchors suitable for being fastened in the sacrum;
· a braid system presenting at least one free end to enable traction to be exerted thereon, and co-operating mechanically with the spinous process of said vertebra; and
· at least one blocking system for blocking said free end of the braid after said traction has been exerted;
said braid system and said blocking system co-operating with each other and with said anchors to provide two traction assemblies between said spinous process and said anchors.

2. A stabilization system according to claim 1, **characterized in that** said braid system comprises two braids, **in that** a first end of each braid is secured to one of said anchors, and **in that** the second end of each braid forms a free traction end engaged in said blocking system.

3. A stabilization system according to claim 2, **characterized in that** said blocking system comprises a body presenting two end faces, with two orifices formed in said body and opening out into the two faces, each orifice being suitable for receiving a respective end of the first and second braid elements, and two movable blocking members suitable for being inserted in the ends of slots opening out into said faces, whereby the braid ends are blocked in the body of the blocking system by wedging.

4. A stabilization system according to claim 2, **characterized in that** said blocking system comprises a body provided with at least one orifice passing right through it, said orifice being suitable for receiving one end of each braid element, said orifice including a shoulder forming a clamping face, and a blocking member that is movable in said orifice, one end of said blocking member forming a second clamping face, the ends of the braid elements being disposed between said clamping faces.

5. A stabilization system according to any one of claims 1 to 4, **characterized in that** it further comprises a first spacer interposed between the sacrum and the spinous process of the fifth lumbar vertebra, and **in that** a portion of one of the braid elements passes over the process of the fifth lumbar vertebra.

6. A stabilization system according to claim 5, **characterized in that** said blocking system is distinct from said first spacer.

7. A stabilization system according to claim 5, **characterized in that** said blocking system is mounted on said first spacer.

8. A stabilization system according to claim 5, **characterized in that** it further comprises a second spacer interposed between the fifth and fourth lumbar vertebrae, said blocking system being mounted on one of said spacers,

9. A stabilization system according to claim 1, **characterized in that** the braid system comprises a single braid, **in that** said blocking system is mechanically connected to one of said anchors, said blocking system co-operating with the free end of the braid, and **in that** the second end of the braid is secured to the second anchor.

10. A stabilization system according to claim 1, **characterized in that** the braid system comprises a single braid, **in that** the stabilization system comprises two blocking systems, each blocking system receiving a free end of said braid, and **in that** each blocking system is mechanically connected to an anchor.

11. A stabilization system according to claim 9 or claim 10, **characterized in that** each blocking system is a self-blocking system serving to block the braid under the effect of the traction exerted on the free end of the braid.

12. A stabilization system according to any one of claims 9 to 11, **characterized in that** each blocking system comprises a body, and **in that** said body is secured to the head of the anchor by a screw fastener member.

13. A stabilization system according to any one of claims 9 to 11, **characterized in that** each blocking system comprises a body that is connected to the head of the anchor via a filamentary element.

14. A stabilization system according to any one of claims 1 to 13, **characterized in that** each anchor comprises a body that is threaded for fastening in the sacrum and a head that is provided with fastener means.

15. A stabilization system according to any one of claims 1 to 13, **characterized in that** said anchor includes extendable means for anchoring in the sacrum.

16. A stabilization system according to any one of claims 1 to 13, **characterized in that** the anchor is an impaction anchor of the staple type.

17. A stabilization system according to any one of claims 1 to 16, **characterized in that** each braid is made of a flexible material or is braided so as to make dynamic stabilization possible.

18. A stabilization system according to any one of claims 9 to 11, **characterized in that** the anchor and the blocking system form a single part.
